# FASCICULE DE BREVET EUROPEEN

(11) **EP 3 452 117 B1**
(45) Date de publication et mention de la délivrance du brevet: **04.08.2021**
(21) Numéro de dépôt: 17725713.6
(22) Date de dépôt: 04.05.2017
(51) Int. Cl.: A61L 15/24, A61L 15/60, A61L 15/34

(54) **COMPOSITION OPTIMISEE POUR PANSEMENT INTERFACE**
OPTIMIERTE ZUSAMMENSETZUNG FÜR INTERFACE-VERBAND
OPTIMIZED COMPOSITION FOR INTERFACE DRESSING

(30) Priorité: 04.05.2016 FR 1654051
(43) Date de publication de la demande: 13.03.2019
(73) Titulaire: Urgo Recherche Innovation et Développement, 21300 Chenove (FR)
(72) Inventeur: AUGUSTE, Stéphane, 21490 Ruffey Les Echirey (FR); DANEROL, Anne-Sophie, 21000 Dijon (FR); DESMAISON, Nadège, 21110 Tart Le Haut (FR); GENOT, Annick, 21000 Dijon (FR); JOLY, Françoise, 21560 Couternon (FR); LABORDE, Aurélie, 21160 Marsannay-La-Cote (FR); GALLAND, Didier, 71270 Navilly (FR); GUILLAMAUD, Christelle, 21300 Chenove (FR)
(74) Mandataire: Cabinet Beau de Loménie
(86) Numéro de dépôt international: PCT/FR2017/051067
(87) Numéro de publication internationale: WO 2017/191416

(56) Documents cités:
- WO-A2-00/16725
- US-A1- 2005 228 116
- US-A1- 2014 341 969

## Description

La présente invention est relative à une nouvelle composition à base de copolymères uniquement tribloc du type ABA, comportant deux blocs terminaux thermoplastiques A styrène et une séquence centrale élastomère B qui est une oléfine saturée, utilisable notamment pour la réalisation d'un pansement interface avec armature, avec support ou autoporté, qui présente une faible affinité vis-à-vis des gants chirurgicaux en latex.

La présente invention est aussi relative à un pansement interface optimisé qui présente une manipulabilité facilitée car il « n'adhère » pas aux gants chirurgicaux en latex grâce à l'utilisation de cette nouvelle composition.

On connaît depuis longtemps le traitement des plaies par des pansements destinés à être mis au contact de la plaie en assurant une interface entre ladite plaie et une compresse absorbante que l'on dépose sur le pansement afin d'absorber les exsudats de la plaie. De tels pansements sont habituellement désignés par l'expression « pansements interface ».

Le pansement commercialisé depuis 2000 par la société Laboratoires URGO sous la dénomination URGOTUL® est un exemple illustratif de tels pansements.

Ce produit est constitué d'une armature faite d'un tissu à mailles ouvertes dont les fils sont enrobés d'un gel cohésif, de façon à laisser les mailles essentiellement non obturées.

Ce gel est formé d'une composition constituée d'une matrice élastomérique hydrophobe à base de copolymères tribloc du type ABA (styrène-oléfine saturée-styrène) fortement plastifiée et contenant en dispersion une faible quantité de particules hydrophiles d'un hydrocolloïde.

Ce pansement et sa composition sont décrits dans l'exemple 1 de la demande de brevet WO 00 16725.

La composition qualitative et quantitative de la matrice élastomérique de ce pansement lui confère des propriétés remarquables en ce qui concerne la favorisation du processus de cicatrisation et en particulier de la prolifération des fibroblastes.

Le produit URGOTUL® présente néanmoins l'inconvénient, dans les cas où l'on souhaite l'appliquer sur des plaies difficilement recouvrables par exemple en raison de leur localisation, de manquer de conformabilité, à cause de la rigidité de son armature.

Pour résoudre ce problème, des pansements interface autoportés ont été décrits dans la demande de brevet FR 2 936 158. Les solutions proposées dans cette demande de brevet permettent d'obtenir des produits qui présentent à la fois une bonne élasticité et une rigidité ainsi qu'une cohésion suffisantes pour pouvoir les manipuler.

La solution proposée dans FR 2 936 158 n'utilise pas de polymères tribloc à séquence centrale saturée mais des composés supplémentaires ou différents de ceux employés dans URGOTUL®.

Or, dans une optique de rentabilité économique, il serait préférable de pouvoir fabriquer un pansement interface autoporté avec des composés identiques ou de même nature que ceux utilisées pour la fabrication du pansement URGOTUL® et de ses déclinaisons.

Ceci présenterait aussi un avantage non négligeable dans le cas où l'on souhaite incorporer dans cette composition des composés actifs, c'est-à-dire, des composés qui ont une action sur le processus de cicatrisation ou le traitement de la plaie, comme par exemple des antibactériens, tels que les sels d'argent, ou des inhibiteurs de MMP (Matrix métalloprotéases) comme le sel de potassium de sucrose octasulfate.

En effet, l'incorporation d'actifs dans ce type de composition est toujours délicate et complexe, chaque composé de la composition pouvant interagir avec les autres ou modifier les propriétés rhéologiques et physico chimiques de la composition voire affecter la stabilité ou la solubilité de l'actif.

En utilisant des composés identiques ou de même nature que ceux déjà utilisés pour la fabrication du pansement URGOTUL® les chances de mettre au point de nouvelles compositions présentant les avantages recherchés sont donc optimisées.

Enfin, l'utilisation de composés identiques ou de même nature que ceux utilisés pour la fabrication du pansement URGOTUL® permettrait de prévenir toute dégradation ou altération, dans les nouvelles compositions recherchées, des propriétés remarquables du produit URGOTUL® sur la prolifération des fibroblastes et sur le processus de cicatrisation.

La demande de brevet FR 2 936 159 décrit une autre variante de pansement interface dans laquelle ce dernier comprend un support, recouvert sur au moins sa face venant au contact de la plaie, d'une composition à base d'une matrice élastomérique hydrophobe l'ensemble ainsi constitué étant perforé pour obtenir des trous traversants. Les compositions utilisées selon ce document sont similaires à celles décrites dans la demande FR 2 936 158 et présentent les mêmes propriétés et inconvénients.

Les demandes de brevet EP 2 524705 et EP 2 524706 décrivent des pansements interface avec armature qui sont réalisés à partir de copolymères uniquement tribloc du type ABA, comportant deux blocs terminaux thermoplastiques A styrène et une séquence centrale élastomère B qui est une oléfine saturée.

Toutefois, les pansements interfaces aujourd'hui commercialisés ou proposés dans ces documents antérieurs souffrent d'une forte affinité vis-à-vis des gants chirurgicaux en latex, que l'on qualifie souvent « d'adhérence » aux gants en latex.

Ainsi, bien que le produit URGOTUL® soit commercialisé depuis 15 ans, ce problème d'affinité ou d'adhérence aux gants en latex n'a jamais pu être résolu.

Les conséquences de ce phénomène sont multiples.

Dans le cas d'un pansement autoporté exempt d'armature, cette affinité vis-à-vis des gants chirurgicaux en latex se traduit par le fait que le pansement interface « colle » à ces derniers en empêchant ainsi de manipuler le pansement et de l'appliquer correctement et rapidement sur la plaie. Le personnel soignant se trouve confronté à un handicap important pour une utilisation rapide et efficace du pansement interface.

Dans le cas d'un pansement avec armature comme le produit URGOTUL®, l'accroche de la composition déposée sur l'armature est faible et celle -ci peut même être transférée sur le gant en latex ce qui rend alors le produit inutilisable.

Pour éviter ce problème, il est préconisé dans la notice du produit URGOTUL® d'humidifier les gants avec du sérum physiologique pour en faciliter la manipulation. Le contact entre les particules d'hydrocolloïde et le sérum physiologique entraine en effet un début de gélification de ces particules qui diminue le phénomène « d'adhérence » aux gants.

Une autre solution possible pour éviter ce problème d'adhérence aux gants est de manipuler le produit uniquement avec des pinces mais à nouveau son utilisation devient complexe, notamment pour certaines localisations de plaies.

Pour obtenir un pansement interface optimal, facile et économique à fabriquer, il serait donc souhaitable de disposer d'une composition pour pansement, qui avec des composés identiques ou de même nature que ceux employés dans URGOTUL® « n'adhère » pas aux gants en latex et ceci aussi bien pour un pansement interface avec armature, que pour un pansement avec support ou autoporté.

La cause de ce phénomène d'affinité vis-à-vis des gants en latex n'a pas pour l'instant été déterminée, mais serait due a priori à la composition de la matrice élastomérique hydrophobe.

L'analyse de l'affinité des matrices élastomériques vis-à-vis des gants en latex est très complexe.

En effet cette « adhérence » peut varier en fonction de la nature des gants en latex ou de leur éventuel traitement en surface. En outre, il est très difficile de mettre au point un test mimant ce phénomène d'adhérence de la composition sur un gant enfilé sur une main. En effet, lorsqu'un gant est enfilé sur une main, sa surface est modifiée en fonction de la morphologie de la main et des doigts, lesquels imposent des rayons de courbure particuliers et appliquent une tension variable sur le gant, ce qui conduit à des surfaces de contact différentes avec la composition. De plus même si le phénomène « d'adhésion » est spectaculaire, les forces à mesurer sont très faibles. Enfin les propriétés d'élasticité des compositions contrarient la mise en œuvre des techniques de mesure de pouvoir adhésif couramment utilisées.

De ce fait, il n'a pas été possible, jusqu'à ce jour, de réaliser des mesures d'adhérence suffisamment précises et fiables pour obtenir des différences significatives permettant de distinguer une composition qui « adhère » d'une composition qui « n'adhère » pas aux gants en latex.

Ceci explique sans doute, au moins en partie, pourquoi aucune solution n'a été proposée depuis environ 15 ans, pour remédier à ce phénomène d'adhérence des pansements interface aux gants en latex.

Après de nombreux échecs, les inventeurs ont réussi à mettre au point une méthode fiable et reproductible d'évaluation de cette affinité en réalisant une mesure de pouvoir adhésif par pelage avec un angle à 90 degrés utilisant des maquettes spécifiques de compositions qui permettent d'obtenir des résultats discriminants avec un gant de référence sur lequel « l'adhérence » du produit URGOTUL® est particulièrement marquée.

Grâce à la mise au point de ce test, les inventeurs ont pu enfin déterminer les paramètres que doit posséder une composition pour présenter une affinité vis-à-vis des gants en latex suffisamment faible pour permettre leur manipulation aisée et pour considérer que cette composition « n'adhère » pas aux gants en latex.

Ainsi, la présente invention a permis de résoudre pour la première fois le problème technique consistant en la mise au point d'une composition spécifique à base de copolymères uniquement tribloc du type ABA, comportant deux blocs terminaux thermoplastiques A styrène et une séquence centrale élastomère B qui est une oléfine saturée, laquelle composition est constituée des composés habituellement employés dans le pansement URGOTUL® et qui grâce à sa faible affinité vis-à-vis des gants chirurgicaux en latex est notamment utilisable à la fois pour la réalisation d'un pansement interface autoporté, d'un pansement avec armature ou avec un support.

Plus précisément, il a été découvert, et ceci constitue le fondement de la présente invention, que des compositions dont la matrice hydrophobe comporte un mélange de 2 copolymères (élastomères) tribloc spécifiques du type styrène - oléfine saturée - styrène, dans lequel le premier copolymère présente une viscosité comprise entre 0,01 et 1 Pa.s telle que mesurée dans une solution à 5 % (masse/masse) dans le toluène et le second copolymère présente une viscosité comprise entre 0,01 et 0,5 Pa.s telle que mesurée dans une solution à 15 % (masse/masse) dans le toluène, ledit mélange de copolymères étant présent au sein de la composition en une quantité pondérale prédéterminée et dans des proportions relatives avec les autres composants de la matrice (plastifiant et vaseline) spécifiquement choisies, permettent de réaliser des pansements qui présentent une affinité suffisamment faible vis-à-vis des gants chirurgicaux en latex pour éviter qu'ils n'y « adhèrent ».

Ainsi selon un premier aspect, la présente invention a pour objet une composition, notamment utile pour la fabrication de pansements, qui présente une faible affinité vis-à-vis des gants en latex, ladite composition comprenant une matrice élastomérique hydrophobe, caractérisé en ce que ladite matrice hydrophobe comprend :
- pour 100 parties en poids d'un mélange P de 2 copolymères tribloc spécifiques du type styrène - oléfine saturée - styrène, un premier qui présente une viscosité comprise entre 0,01 et 1 Pa.s telle que mesurée à 30°C dans une solution à 5 % (masse/masse) dans le toluène et un second qui présente une viscosité comprise entre 0 ,01 et 0,5 Pa.s telle que mesurée à 30°C dans une solution à 15 % (masse/masse) dans le toluène;
- de 300 à 1000 parties en poids d'un plastifiant H, de préférence une huile plastifiante ; et
- de 90 à 600 parties en poids de vaseline V ;
étant en outre précisé que :
- la quantité totale, représentée par P+H+V, de mélange d'élastomères, du plastifiant et de la vaseline est comprise entre 490 et 1700 parties en poids ;
- le rapport entre la quantité totale du mélange d'élastomères, du plastifiant et de la vaseline et la quantité de vaseline, représenté par P+H+V/V, est inférieur à 11 ; et ledit mélange de 2 copolymères comprend au moins 20 % en poids du premier copolymère.

Selon une caractéristique particulière, la matrice hydrophobe précitée comprend en outre des particules d'hydrocolloïdes en une quantité inférieure ou égale à 25 % en poids, rapportée au poids total de ladite matrice hydrophobe.

Selon un second aspect, la présente invention a pour objet un pansement interface avec armature, avec support ou autoporté qui comprend une telle composition.

La composition selon l'invention qui présente une faible affinité vis-à-vis des gants chirurgicaux en latex, comprend une association de 2 copolymères uniquement tribloc du type ABA appartenant respectivement à des catégories prédéterminées.

Les copolymères séquencés utilisés dans le cadre de l'invention sont des copolymères triblocs du type ABA comportant deux blocs terminaux thermoplastiques A styrène et une séquence centrale élastomère B qui est une oléfine saturée. Les séquences B d'oléfines saturées sont par exemple des séquences éthylène-butylène, éthylène-propylène ou éthylène-éthylène-propylène.

Par souci de simplicité, dans la présente description, les blocs polymériques constituant les copolymères précités sont désignés par la nature de leurs unités récurrente. Ainsi, l'expression « bloc » ou « séquence styrène A » désigne une séquence poly(styrène) et l'expression « bloc » ou « séquence oléfine saturée » désigne une séquence poly(oléfine saturée).

Les copolymères uniquement triblocs à séquence centrale saturée sont bien connus de l'homme de l'art et sont par exemple commercialisés :
- par la société KRATON POLYMERS sous la dénomination KRATON® G, et en particulier les grades KRATON® G1651, KRATON® G1654, KRATON® G 1657, KRATON® G1652 ou KRATON® G1650 et par la société KURARAY sous les dénominations SEPTON® et en particulier les grades 8006 ou 8004 pour les copolymères séquencés poly (styrène-éthylène-butylène-styrène) (en abrégé SEBS);
- par la société KURARAY sous la dénomination SEPTON® pour les copolymères séquencés poly (styrène-éthylène-propylène-styrène) (en abrégé SEPS) et en particulier les grades 2005, 2006 ou 2063 et pour les polymères séquencés poly (styrène-éthylène-éthylène-propylène-styrène) (en abrégé SEEPS) et en particulier les grades 4033, 4044, 4055, 4077 ou 4099.

Dans le cadre de la présente invention, on préférera les copolymères triblocs SEBS, SEPS ou SEEPS ayant une teneur en styrène comprise entre 25 et 45 % en poids par rapport au poids dudit copolymère SEBS, SEPS ou SEEPS.

Parmi les copolymères qui présentent une viscosité comprise entre 0,01 et 1 Pa.seconde mesurée dans une solution à 5% (masse/masse) dans le toluène, on peut citer les copolymères commercialisé par la société KRATON sous les grades KRATON® G 1651 et KRATON® G 1654 et les copolymères commercialisés par la société KURARAY sous les grades SEPTON® 2005, 2006, 8006, 4044, 4055, 4077 ou 4099.

Parmi les copolymères qui présentent une viscosité comprise entre 0,01 et 0,5 Pa.seconde mesurée dans une solution à 15% (masse/masse) dans le toluène on peut citer les copolymères commercialisés par la société KRATON sous les grades KRATON® G 1650, KRATON® G 1657 et KRATON® G 1652 et les copolymères commercialisés par la société KURARAY sous les grades SEPTON® 2063 ou 4033.

Ces viscosités sont mesurées à 30°C à l'aide d'un viscosimètre Brookfield modèle LTV dans une solution dans le toluène à 5 % ou 15 % masse/masse en fonction du poids moléculaire du copolymère.

D'une façon générale, ce mélange de 2 copolymères comprendra au moins 20% en poids du copolymère qui présente une viscosité comprise entre 0,01 et 1 Pa.seconde mesurée dans une solution à 5 % masse/masse dans le toluène.

De préférence cette proportion sera supérieure à 30%. En particulier pour la réalisation de pansements interfaces autoporté, on préférera employer des mélanges qui comprennent au moins 50% et de préférence au moins 70 % de copolymère qui présente une viscosité comprise entre 0,01 et 1 Pa.s pour obtenir non seulement une faible affinité vis-à-vis des gants en latex mais en outre les meilleures propriétés de cohésion et d'élasticité.

De façon générale, la quantité du mélange de ces 2 copolymères dans la composition finale pourra être comprise entre 5 et 20 % en poids, de préférence entre 5 et 15 % et plus particulièrement entre 7 et 10% en poids, rapportée au poids total de la composition.

Contrairement à ce qu'on aurait pu attendre il a été observé que le mélange de ces copolymères, dans les proportions définies ci- dessus, conduit à des compositions homogènes et qui présentent de bonnes propriétés de cohésion, malgré l'utilisation de 2 copolymères tribloc aux viscosités si différentes.

Dans le cadre de la présente invention on préférera tout particulièrement l'utilisation de deux copolymères séquencés SEBS, et en particulier l'association des copolymères KRATON® G 1654 et KRATON® G 1650 dans laquelle la répartition KRATON® G 1654 / KRATON® G 1650 en pourcentage pondéral est comprise entre 50/50 et 70/ 30, en une quantité totale qui représente de préférence entre 5 et 15 % et de préférence entre 7 et 10 % en poids, rapportée au poids total de la composition.

Afin de réaliser des pansements interface le mélange de ces 2 copolymères est associé à un (ou plusieurs) composé(s) plastifiants et à de la vaseline pour former une matrice hydrophobe.

Pour obtenir des compositions qui présentent une faible affinité vis-à-vis des gants en latex, cette matrice hydrophobe comprend obligatoirement ces trois types de constituants (mélange de copolymères, plastifiant et vaseline), en des proportions spécifiques.

Dans le cadre de l'invention, la vaseline est une vaseline conforme à la Pharmacopée Française disponible commercialement.

Les plastifiants susceptibles d'être utilisés sont bien connus et destinés à améliorer les propriétés d'étirement, de souplesse, d'extrudabilité ou de mise en œuvre des copolymères. On pourra utiliser à cet effet un ou plusieurs plastifiants si nécessaire.

D'une façon générale, en tant que plastifiants, on préférera des composés liquides, compatibles avec la séquence centrale oléfine saturée des copolymères séquencés précités, et plus particulièrement les composés qui présentent un point de goutte inférieur ou égal à 35°C.

Parmi les composés plastifiants susceptibles d'être utilisés à cet effet, on citera en particulier les huiles minérales plastifiantes.

Alternativement, on peut aussi utiliser des produits de synthèse à base de mélanges liquides d'hydrocarbures saturés comme par exemple les produits commercialisés par la société TOTAL sous la dénomination GEMSEAL® et en particulier le produit GEMSEAL® 60 qui est un mélange isoparaffinique issu d'une coupe pétrolière totalement hydrogénée.

Dans le cadre de la présente invention, on utilisera de préférence des huiles plastifiantes et en particulier des huiles minérales formées de composés de nature paraffinique ou naphténique, ou de leurs mélanges, dans des proportions variables.

Des huiles minérales plastifiantes particulièrement préférées sont formées de mélanges de composés de nature paraffinique et naphténique, et en particulier de tels mélanges dans lesquels la proportion de composés de nature paraffinique est majoritaire.

Parmi les huiles plastifiantes convenant particulièrement, on peut citer les produits commercialisés par la société SHELL sous les dénominations ONDINA® et en particulier ONDINA® 919 ou l'huile commercialisée par la société PETRO CANADA sous la référence PURETOL® 9D.

Comme indiqué précédemment, les quantités de plastifiant et de vaseline doivent être choisies en fonction de la quantité totale d'élastomère constitué par le mélange précité de copolymères.

Sur la base du test qui sera décrit ultérieurement, on considère qu'une composition qui présente un pouvoir adhésif inférieur ou égal à 7cN/cm mesuré sur un gant commercialisé par la société VWR sous la référence 112-1567, « n'adhère » pas aux gants en latex.

Pour obtenir ce résultat il a été déterminé que la matrice hydrophobe doit comprendre :
- pour 100 parties en poids d'un mélange P de 2 copolymères tribloc spécifiques du type styrène - oléfine saturée - styrène, un premier qui présente une viscosité comprise entre 0,01 et 1 Pa.s telle que mesurée à 30°C dans une solution à 5 % (masse/masse) dans le toluène et un second qui présente une viscosité comprise entre 0,01 et 0,5 Pa.s mesurée à 30°C dans une solution à 15 % (masse/masse) dans le toluène ;
- de 300 à 1000 parties en poids d'un plastifiant H, de préférence une huile plastifiante ; et
- de 90 à 600 parties en poids de vaseline V ;
étant en outre précisé que :
- la quantité totale représentée par P+H+V de mélange de copolymères, du plastifiant et de la vaseline est comprise entre 490 et 1700 parties en poids ;
- le rapport entre la quantité totale du mélange de copolymères, du plastifiant et de la vaseline et la quantité de vaseline représenté par P+H+V/V est inférieur à 11 ; et ledit mélange de 2 copolymères comprend au moins 20 % en poids du premier copolymère.

Dans ce qui suit, et par souci de simplicité :
- l'expression « la quantité totale représentée par P+H+V de mélange de copolymères, du plastifiant et de la vaseline est comprise entre X et Y parties en poids » sera parfois remplacée par l'équation X<P+H+V<Y ; et
- l'expression « le rapport entre la quantité totale du mélange de copolymères, du plastifiant et de la vaseline et la quantité de vaseline représenté par P+H+V/V est inférieur à Z » sera parfois remplacée par l'équation P+H+V/V<Z ;
étant précisé que X, Y et Z désignent des nombres entiers.

Pour la réalisation de pansements interface autoportés présentant le meilleur compromis entre élasticité et cohésion, on préférera une composition dont la matrice hydrophobe comprend :
- pour 100 parties en poids d'un mélange P de 2 copolymères tribloc spécifiques du type styrène - oléfine saturée - styrène, un premier qui présente une viscosité comprise entre 0,01 et 1 Pa.s telle que mesurée à 30°C dans une solution à 5 % (masse/masse) dans le toluène et un second qui présente une viscosité comprise entre 0,01 et 0,5 Pa.s mesurée à 30°C dans une solution à 15 % (masse/masse) dans le toluène;
- de 500 à 1000 parties en poids d'un plastifiant H, de préférence une huile plastifiante ; et
- de 100 à 400 parties en poids de vaseline V ;
étant en outre précisé que :
les équations suivantes sont satisfaites :
700 < P+H+V< 1200 et
(P+H+V) / V < 8
et ledit mélange de 2 copolymères comprend au moins 30 % en poids, du premier copolymère.

Selon un mode de réalisation actuellement préféré de la présente invention, qui permet d'obtenir les pansements interface les plus performants et la composition qui présente la plus faible affinité vis-à-vis des gants en latex, la matrice hydrophobe comprend :
- pour 100 parties en poids d'un mélange P de 2 copolymères tribloc spécifiques du type styrène - oléfine saturée - styrène, un premier qui présente une viscosité comprise entre 0,01 et 1 Pa.s telle que mesurée à 30°C dans une solution à 5 % (masse/masse) dans le toluène et un second qui présente une viscosité comprise entre 0,01 et 0,5 Pa.s mesurée à 30°C dans une solution à 15 % (masse/masse) dans le toluène ;
- de 650 à 800 parties en poids d'un plastifiant H, de préférence une huile plastifiante ; et
- de 150 à 200 parties en poids de vaseline V ;
étant en outre précisé que :
les équations suivantes sont satisfaites :
900 < P+H+V< 1100 et
(P+H+V) / V < 6
et ledit mélange de 2 copolymères comprend au moins 50 % en poids du premier copolymère.

Sur la base du test mis au point par les inventeurs de telles compositions présentent un pouvoir adhésif inférieur ou égal à 3 cN/cm, mesuré sur un gant commercialisé par la société VWR sous la référence 112-1567, et sont particulièrement bien adaptées pour la réalisation d'un pansement interface autoporté facile à manipuler avec des gants chirurgicaux en latex.

Ces compositions sont également utiles pour la préparation de pansements avec armature ou avec support.

La matrice hydrophobe qui vient d'être décrite constitue l'élément essentiel des compositions permettant d'obtenir une composition et un pansement qui présentent une faible affinité vis-à-vis des gants en latex conformément à l'invention.

De telles compositions peuvent néanmoins comprendre des composés additionnels et en particulier des composés choisis parmi les antioxydants, les hydrocolloïdes, et les agents actifs ou adjuvants couramment utilisés dans le domaine du traitement des plaies.

Par « agents antioxydants », on entend désigner ici les composés couramment employés par l'homme de l'art pour assurer la stabilité des composés entrant dans la formulation des compositions, en particulier vis-à-vis de l'oxygène, la chaleur, l'ozone ou les rayonnements ultra-violets.

Comme exemples d'agents antioxydants appropriés, on peut citer notamment les antioxydants phénoliques comme en particulier les produits commercialisés par la société BASF sous les dénominations IRGANOX® 1010, IRGANOX® 565, IRGANOX® 1076.

D'une façon générale ces agents antioxydants pourront être utilisés seuls ou en association en une quantité de l'ordre de 0,05 à 1 % en poids, de préférence de 0,05 à 0,2 % en poids, rapportée au poids total de la composition.

Dans le cadre de la présente invention, on préférera l'utilisation du produit IRGANOX® 1010 en une quantité comprise entre 0,05 et 0,2 % en poids, rapportée au poids total de la composition.

Selon un autre mode de réalisation de l'invention particulièrement préféré dans le cadre de la réalisation de pansements interface autoportés, avec support ou avec armature pour la cicatrisation des plaies, les compositions selon l'invention comprennent des particules hydrophiles d'un hydrocolloïde (ou particules d'hydrocolloïde).

Ces particules permettent en effet le retrait indolore d'un pansement interface et le maintien d'un milieu humide au niveau de la plaie afin de favoriser la cicatrisation.

A cet effet, une quantité faible de particules hydrophiles d'un hydrocolloïde est ainsi soit disposée en surface de la matrice hydrophobe soit, de préférence, dispersée de façon homogène au sein de la composition.

Par « hydrocolloïde » ou « particules d'hydrocolloïde », on entend désigner ici tout composé habituellement utilisé par l'homme de l'art pour son aptitude à absorber les liquides aqueux tels que l'eau, le sérum physiologique ou les exsudats d'une plaie.

Comme hydrocolloïdes appropriés, on peut citer par exemple la pectine, les alginates, les gommes végétales naturelles comme en particulier la gomme de Karaya, les dérivés de cellulose tels que les carboxyméthylcelluloses et leurs sels de métal alcalin tels que le sodium ou le calcium, ainsi que les polymères synthétiques à base de sels de l'acide acrylique, connus sous l'appellation "superabsorbants", comme par exemple les produits commercialisés par la société BASF sous la dénomination LUQUASORB® 1003 ou par la société CIBA Specialty Chemicals sous la dénomination SALCARE® SC91 ainsi que les mélanges de ces composés.

Certains de ces superabsorbants qualifiés de « microcolloïdes » car ils présentent une taille de particules inférieure à 10 micromètres peuvent bien entendu être également utilisés.

Les hydrocolloïdes préférés dans le cadre de la présente invention sont les sels de métal alcalin de la carboxyméthylcellulose, et en particulier la carboxyméthylcellulose de sodium (CMC).

La taille des particules d'hydrocolloïde est généralement comprise entre 50 et 100 microns, avantageusement de l'ordre de 80 microns.

D'une façon générale, la quantité de particules d'hydrocolloïde incorporées dans la composition selon l'invention sera avantageusement inférieure ou égale à 25 % en poids, rapportée au poids total de ladite matrice hydrophobe.

Si les particules d'hydrocolloïde sont disposées en surface de la matrice hydrophobe, leur quantité sera de préférence de l'ordre de 1 à 10% et plus particulièrement de 2 à 5% en poids, rapportée au poids total de ladite matrice hydrophobe.

Les particules d'hydrocolloïde sont de préférence dispersées de façon homogène au sein de la composition.

La quantité de particules d'hydrocolloïde dispersées dans la composition sera dans ce cas avantageusement de l'ordre de 2 à 20% en poids, de préférence de 5 à 18% en poids, de préférence encore de 10 à 15% en poids rapportée au poids total de la matrice hydrophobe.

Le choix d'une quantité de particules d'hydrocolloïde comprise dans ces plages de valeurs est importante pour la réalisation d'un pansement interface, et en particulier un pansement interface autoporté aéré, afin d'éviter que la gélification de la composition n'entraine la fermeture des trous traversants lors de l'absorption des exsudats.

Selon un autre mode de réalisation, les compositions selon l'invention comprennent un ou plusieurs adjuvants et /ou agents actifs couramment utilisés dans le domaine du traitement des plaies ou dans le domaine pharmacologique.

La composition peut ainsi contenir des agents actifs ayant un rôle favorable dans le traitement des plaies et notamment capables d'induire ou d'accélérer la cicatrisation pendant la phase de détersion et/ou de granulation de la plaie.

La présence d'hydrocolloïdes au sein de la composition favorisera le relargage de ces agents actifs.

Ces agents actifs pourront être utilisés en une quantité de l'ordre de 0,01 à 20 % en poids, de préférence de 1 à 15 % en poids, et de préférence encore de 2 à 10 % en poids, rapportée au poids total de la composition.

Parmi les agents actifs susceptibles d'être utilisés dans le cadre de l'invention, on citera, pour exemple, les agents bactéricides ou bactériostatiques, les agents favorisant la cicatrisation, les agents anti-douleurs ou les anesthésiques locaux ainsi que les agents anti-inflammatoires.

A titre d'adjuvants susceptibles d'être utilisés dans les compositions selon l'invention, on peut citer des composés connus pour favoriser le relargage des agents actifs, comme par exemple les produits Montanox® 80 ou Sepinov® EMT 10 qui sont couramment utilisés dans les produits URGOTUL® qui incorporent des agents actifs.

Ces adjuvants pourront être utilisés en une quantité de l'ordre de 1 à 15% rapportée au poids total de la composition.

Bien évidemment les modes de réalisation particuliers qui viennent d'être décrits peuvent être mis en œuvre séparément ou selon l'une quelconque de leurs combinaisons.

Les compositions selon l'invention permettent en particulier de réaliser des pansements interface autoportés, avec une armature ou avec un support.

Dans le cadre de la réalisation d'un pansement interface on préférera l'utilisation d'une composition qui comprend des composés (copolymères, huile minérale, vaseline, antioxydant et hydrocolloïdes) identiques à, ou de même nature que, ceux utilisés dans le produit URGOTUL®.

Afin de réaliser un pansement, les compositions selon l'invention seront formées en couche mince, avec des trous traversants, disposés de préférence de façon répartie dans ladite couche.

Les trous traversants peuvent être réalisés par perforation ou poinçonnage d'une composition préalablement formée en couche mince, seule ou associée à un support provisoire ou à un film protecteur habituellement utilisé pour la fabrication de pansement, ou bien encore par une enduction tramée sur un support provisoire.

Alternativement, les pansements conformes à l'invention peuvent être fabriqués par coulage à chaud d'une composition telle que décrite précédemment sur une plaque gravée avec le motif retenu pour former des trous traversants, suivi d'un refroidissement et d'un démoulage.

D'une façon générale, les pansements conformes à l'invention présenteront une épaisseur comprise entre 0,4 mm et 2 mm, de préférence entre 0,5 mm et 1 mm, de préférence encore de l'ordre de 0,6 mm.

Les trous traversants peuvent être de géométrie quelconque et présenteront par exemple une section transversale circulaire, rectangulaire, trapézoïdale ou carrée.

Leur surface sera généralement comprise entre 0,25 et 5 mm².

Ces trous présenteront notamment un diamètre moyen compris entre 0,5 et 2 mm, de préférence de l'ordre de 1 mm, lorsque leur section transversale est circulaire.

Ces trous seront répartis, de préférence de façon régulière, avec une densité telle que la surface totale des trous représente entre 20 et 70%, et de préférence entre 30 et 50% de la surface totale du pansement.

Selon un mode de réalisation préféré, le pansement interface autoporté selon l'invention se présente sous la forme d'un filet aéré (ou grille) de préférence de maille carrée présentant :
- une épaisseur du filet comprise entre 0,5 et 2 mm ;
- une « largeur de fil » (largeur de l'espace entre deux trous consécutifs) comprise entre 1 et 10 mm, et de préférence entre 1 et 5 mm ;
- un grammage compris entre 200 et 1700 g/m², et de préférence compris entre 300 et 800 g/m².

Selon un mode de réalisation particulièrement préféré de l'invention, un tel pansement se présentera sous la forme d'un filet aéré à maille carrée présentant :
- une épaisseur du filet de 600 microns environ ;
- une largeur de fil (ou taille de maille) de l'ordre de 2 mm ;
- un grammage de l'ordre de 450 g/m².

Pour la réalisation de tels pansements autoportés on pourra se référer pour plus de détails à la demande de brevet FR 2 936 158.

Selon un mode de réalisation actuellement préféré, la présente demande vise à couvrir un pansement interface autoporté comprenant une couche mince possédant des trous traversants pour laisser passer les exsudats caractérisé en ce que la composition de la matrice hydrophobe comprend :
- pour 100 parties en poids d'un mélange P de 2 copolymères tribloc spécifiques du type styrène - oléfine saturée - styrène, un premier qui présente une viscosité comprise entre 0,01 et 1 Pa.s telle que mesurée à 30°C dans une solution à 5% (masse/masse) dans le toluène et un second qui présente une viscosité comprise entre 0,01 et 0,5 Pa.s mesurée à 30°C dans une solution à 15% (masse/masse) dans le toluène ;
- de 650 à 800 parties en poids d'un plastifiant H, de préférence une huile plastifiante ; et
- de 150 à 200 parties en poids de vaseline V ;
étant en outre précisé que :
les équations suivantes sont satisfaites :
900 < P+H+V< 1100 et
(P+H+V) / V < 6
ledit mélange de 2 copolymères comprend au moins 50 % en poids du premier copolymère ;
et des particules d'hydrocolloïde en une quantité comprise entre 10 et 20 % en poids rapportée au poids total de la matrice hydrophobe.

Les techniques de fabrication d'un pansement interface avec armature ou avec support sont aussi bien connues de l'homme de l'art et on pourra par exemple se référer aux procédés décrits dans les demandes de brevet WO 00 16725 et FR 2 936 159.

Afin de protéger la composition de l'environnement extérieur, le pansement interface sera recouvert de préférence sur chacune de ses faces, par un film protecteur provisoire qui sera retiré avant usage par l'utilisateur.

Afin de faciliter encore la manipulation du pansement interface, en particulier s'il est autoporté, on pourra substituer ces deux protecteurs provisoires par un protecteur unique tel que décrit dans la demande de brevet WO 2008 / 145 884 dont la structure particulière facilite l'application du pansement sur la plaie.

Bien entendu les compositions selon l'invention pourront être aussi utilisées dans la fabrication de tout produit, et en particulier tout dispositif médical qui est susceptible, lors de son utilisation, d'être manipulé avec des gants en latex.

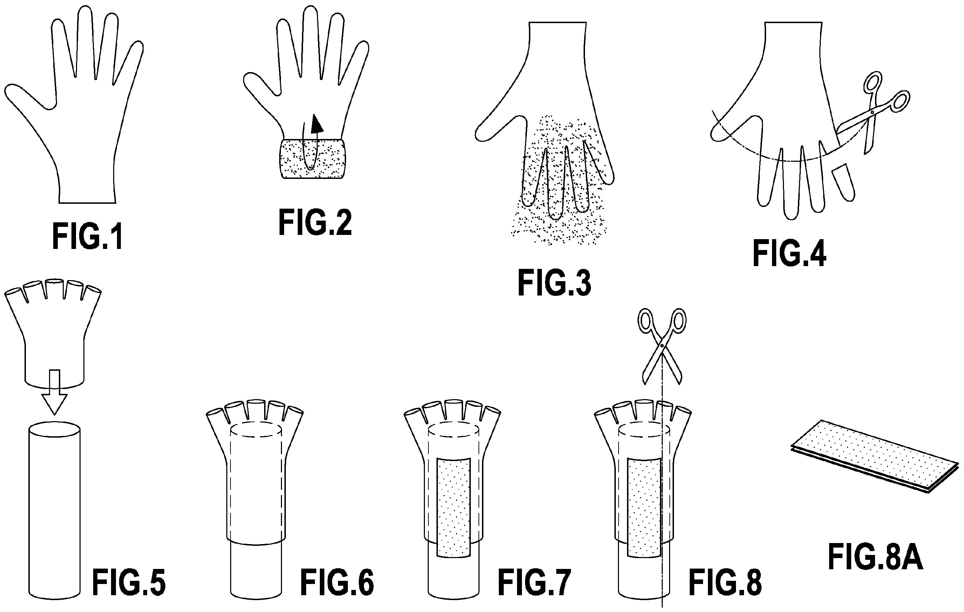

L'invention va maintenant être illustrée par les exemples et les tests suivants, en référence aux dessins annexés dans lesquels les figures 1 à 13 illustrent les différentes étapes du test mis au point par les inventeurs pour mesurer l'adhérence aux gants en latex.

### Préparation des compositions

Les compositions des exemples 1 à 7 ont été élaborées à l'aide des constituants suivants dans les proportions, exprimées en pourcentage en poids, mentionnées dans le tableau 1.
- Elastomère : copolymère séquencé de poly (styrène-éthylène-butylène-styrène) (en abrégé SEBS) :
- KRATON® G 1651 viscosité à 5% (masse/ masse) dans le toluène : 0,04Pa.s
- KRATON® G 1654 viscosité à 5% (masse/masse) dans le toluène : 0,02Pa.s
- KRATON® G 1652 viscosité à 15% (masse/masse) dans le toluène : 0,088Pa.s
- KRATON® G 1650 vicosité à 15% (masse / masse) dans le toluène : 0,2Pa.s
   - plastifiant : huile minérale Ondina® 919 commercialisée par la société SHELL
   - anti-oxydant : IRGANOX® 1010 commercialisé par la société BASF
   - vaseline : vaseline Codex® A commercialisée par la société AIGLON
   - hydrocolloïde : Carboxyméthylcellulose sodique CMC BLANOSE® 7H4XF commercialisée par la société ASHLAND.

### Fabrication de la composition

Dans un mélangeur IKA, on a introduit successivement sous agitation à une température de consigne de 90°C le plastifiant, l'hydrocolloïde et la vaseline et on a malaxé jusqu'à l'obtention d'un mélange homogène.

Après avoir porté la température de consigne à 140°C, on a introduit sous agitation les 2 copolymères et l'anti-oxydant, puis on a malaxé jusqu'à l'obtention d'un mélange homogène.

On a ensuite laissé refroidir, puis on a vidangé le mélangeur.

**Tableau 1**

| | Exemple 1 | Exemple 2 | Exemple 3 | Exemple 4 | Exemple 5 | Exemple 6 | Exemple 7 |
|---|---|---|---|---|---|---|---|
| plastifiant | 74,95 | 75 | 75 | 61,5 | 68,48 | 68,48 | 68,48 |
| vaseline | 5 | | 4,95 | 15 | 8,02 | 8,02 | 8,02 |
| antioxydant | 0,12 | 0,2 | 0,12 | 0,2 | 0,2 | 0,2 | 0,2 |
| hydrocolloïde | 15 | 10 | 15 | 15 | 15 | 15 | 15 |
| Kraton® G 1651 | 4,93 | | 2,96 | | | | |
| Kraton® G 1654 | | 7,8 | | 5,7 | 4,15 | 5,7 | 2,6 |
| Kraton® G 1650 | | 7 | | 2,6 | 4,15 | 2,6 | 5,7 |
| Kraton® G 1652 | | | 1,97 | | | | |

### Mise en évidence de l'adhérence des compositions aux gants en latex :

Les conditions de réalisation du test et des maquettes utilisées étaient les suivantes.

### Réalisation des maquettes

Les maquettes se présentaient généralement sous la forme de compositions renforcées dans lesquelles une armature est noyée afin de les rigidifier.

L'armature utilisée était un tricot thermofixé, en fils polyester, fabriqué par la société MDB TEXINOV sous la référence 555. Son grammage était de 40 g/m². Elle présentait une extensibilité, mesurée selon la norme EN 13726-4, de 2,7 N/cm dans le sens transversal et 24 N/cm dans le sens longitudinal.

On a réalisé ainsi 2 plaques à l'aide des compositions à tester et on a incorporé cette armature entre les 2 plaques de composition, en appliquant une forte pression à l'aide d'une presse hydraulique sur l'ensemble selon le protocole suivant.

On a préchauffé les 2 plateaux de la presse. Sur le plateau inférieur de la presse on a déposé un film plastique antiadhérent, par exemple un film de polyester siliconé - fluoré, référencé 50 MD 07, blanc commercialisé par la société Siliconature ; la face siliconée - fluorée étant disposée de façon opposée au plateau inférieur. On a étalé sur cette face 10 g d'une des compositions décrites dans le tableau 1 et on a recouvert cette dernière par un film de polyester siliconé de 75 micromètres d'épaisseur, le côté siliconé étant disposé au contact de la composition. On a placé 2 cales de 0,70 mm entre les 2 films de polyester aux extrémités du plateau inférieur de la presse et on a soumis l'ensemble à une pression de 200 bars et une température de l'ordre de 90 à 100 C°.

On a fabriqué une seconde plaque de la même façon.

Sur l'une de ces plaques, après avoir retiré le polyester siliconé- fluoré, on a appliqué un échantillon de l'armature précédemment définie sur la composition apparente.

Partant de l'autre de ces plaques, après avoir retiré le polyester siliconé- fluoré, on a appliqué la face de cette plaque, comportant la composition à tester, sur l'armature associée en surface à l'autre plaque, pour obtenir ainsi un complexe polyester siliconé - composition - armature - composition - polyester siliconé.

On a placé 2 cales de 1mm et 0,2mm respectivement entre les 2 films de polyester siliconés aux extrémités du plateau inférieur de la presse et on a appliqué une pression de 200 bars et une température de l'ordre de 90 à 100C°.

On a laissé refroidir la maquette et on a contrôlé son épaisseur avec un micromètre de manière à obtenir une maquette dont l'épaisseur est de l'ordre de 1 à 1,1mm. Il est en effet nécessaire d'utiliser de telles maquettes de faible épaisseur, pour mettre en œuvre le test permettant de mesurer l'adhérence aux gants de latex.

### Mesure de l'adhérence aux gants en latex

On a mesuré le pouvoir adhésif des maquettes réalisées selon le protocole décrit précédemment, à l'aide d'un test de pelage à angle droit classique. Ce test de pelage a été réalisé avec un dynamomètre MTS équipé d'un capteur de 2 newtons. Ce dynanomètre permet de mesurer la force nécessaire pour décoller la maquette à tester d'une plaque en acier, recouverte d'un échantillon de gant en latex. Cette force est exprimée cN/cm.

Le test a été réalisé à 23 C° et 50 % d'humidité relative.

Le mode opératoire du test était le suivant.

### a. Préparation de la plaque en acier recouverte d'un échantillon de gant en latex.

Cette préparation est illustrée par les figures 1 à 13 qui décrivent les étapes successives de réalisation.

On utilise un gant en latex (taille 8-9) référencé 112- 1567 commercialisé par la société VWR. (Figure 1).

On retourne le gant en latex poudré (figure 2) en ramenant vers l'intérieur la face utilisateur. On secoue le gant ainsi retourné pour éliminer la poudre (figure 3) et on coupe les doigts et le pouce du gant (figure 4). On enfile le gant, toujours retourné, sur une éprouvette de 1 litre de 68 mm de diamètre (figures 5 et 6) la face utilisateur du gant venant ainsi en contact avec l'éprouvette. On colle sur le gant une bande de masking (commercialisée par la société Plasto sous la référence P 3650) de 5 cm de largeur et de 10 cm de longueur en évitant la création de plis. On découpe le gant en suivant les bords du masking pour obtenir un échantillon recouvert du masking sur une de ses faces (figures 8 et 8A).

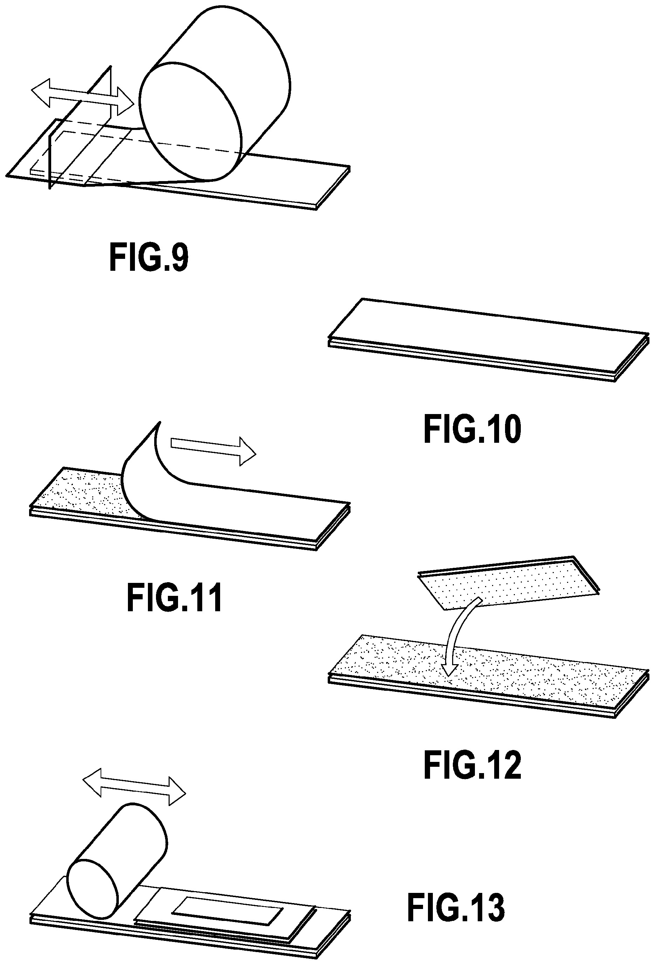

Sur la plaque d'acier utilisée pour réaliser le test de pelage on colle un adhésif double face commercialisé par la société Plasto sous la référence P753 en évitant la création de plis ou de bulles (figures 9 et 10). On décolle alors le protecteur de l'adhésif double face (figure 11) et on vient coller sur l'adhésif la face masking de l'échantillon de gant obtenu précédemment en évitant les plis et les bulles (figure 12). On découpe une éprouvette de la maquette à tester de 1,5 cm de largeur et de 10 cm de longueur que l'on dépose en la centrant sur le gant fixé à la plaque d'acier tel qu'obtenu précédemment et on passe en 2 allers- retours un rouleau de 3 kg à la vitesse de 200 mm/mn (figure 13).

On a procédé alors au test de pelage à 90 degrés à la vitesse de 300 mm/mn. On a reproduit le test 5 fois pour chaque composition à tester. Les résultats obtenus sont la moyenne de ces 5 mesures. Les pouvoirs adhésifs obtenus exprimés avec leur écart - type (i) sont rassemblés dans le tableau 2.

**Tableau 2**

| | Exemple 1 | Exemple 2 | Exemple 3 | Exemple 4 | Exemple 5 | Exemple 6 | Exemple 7 |
|---|---|---|---|---|---|---|---|
| Pouvoir adhésif | 9,76 i=1,89 | 17,36 i=3,62 | 47,12 i=7,96 | 2,08 i=0,55 | 3,72 i=0,74 | 4,7 i=1,59 | 3,96 i=3,62 |

Les exemples 4 à 7 correspondent à des compositions selon l'invention.

En outre avec le gant en latex sélectionné il a été constaté que toutes les maquettes correspondant aux exemples 4 à 7 selon l'invention étaient facilement manipulables.

Les compositions des exemples 1 et 3 correspondent respectivement aux formulations des exemples 1 et 5 de la demande de brevet WO 00 / 16725. Quand on a manipulé les maquettes qui correspondent à ces 2 exemples et celle qui correspond à l'exemple 2, avec le gant en latex sélectionné on a constaté que toutes ces maquettes « adhéraient » à ce gant en latex ce qui les rendait très difficilement manipulables.

On a bien la confirmation que le test effectué est représentatif du phénomène d'affinité. Quand le pouvoir adhésif, mesuré selon ce test, est inférieur à 7cN/cm les maquettes « n'adhèrent » pas aux gants en latex.

Aucune des compositions des exemples comparatifs 1 à 3 ne répond à ce critère de "non-adhérence".

On a par ailleurs réalisé un pansement interface autoporté à l'aide de la composition de l'exemple 4 qui présente le plus faible pouvoir adhésif trouvé, selon le mode opératoire suivant.

Dans un mélangeur vertical, on a introduit successivement sous agitation à une température de consigne de 90°C le plastifiant et l'hydrocolloïde et on a malaxé jusqu'à l'obtention d'un mélange homogène.

Après avoir porté la température de consigne à 140°C, on a introduit sous agitation les 2 copolymères et l'anti-oxydant, puis on a malaxé jusqu'à l'obtention d'un mélange homogène.

La vaseline a alors été introduite sous agitation à 140°C en deux fois jusqu'à l'obtention d'un mélange homogène.

Le mélange ainsi obtenu a été coulé à chaud à une température de l'ordre de 120-130°C sur une plaque plane gravée formant l'empreinte d'un filet ou grille à maille carrée.

Après refroidissement et démoulage, on a obtenu le pansement attendu sous forme d'un filet à mailles carrées présentant une épaisseur de 600 µm environ, une taille de maille de l'ordre de 2 mm et un grammage de l'ordre de 450 g/m².

Les pansements ainsi réalisés ont été placés entre deux films protecteurs provisoires en polyester siliconé de 50 µm d'épaisseur.

On a ainsi obtenu un pansement interface autoporté qui présente d'excellentes propriétés de cohésion et d'extensibilité mais surtout qui ne pose aucun problème lors de sa manipulation avec des gants en latex car il n'y « adhère » pas.

## Revendications

1. Composition qui présente une faible affinité vis-à-vis des gants en latex comprenant une matrice hydrophobe, **caractérisée en ce que** ladite matrice hydrophobe comprend :
- pour 100 parties en poids d'un mélange P de 2 copolymères tribloc du type styrène - oléfine saturée - styrène, un premier qui présente une viscosité comprise entre 0,01 et 1 Pa.s telle que mesurée à 30°C dans une solution à 5 % (masse/masse) dans le toluène et un second qui présente une viscosité comprise entre 0,01 et 0,5 Pa.s mesurée à 30°C dans une solution à 15 % (masse/masse) dans le toluène ;
- de 300 à 1000 parties en poids d'un plastifiant H, de préférence une huile plastifiante ; et
- de 90 à 600 parties en poids de vaseline V ;
étant en outre précisé que :
- la quantité totale, représentée par P+H+V, de mélange d'élastomères, du plastifiant et de la vaseline est comprise entre 490 et 1700 parties en poids ;
- le rapport entre la quantité totale du mélange d'élastomères, du plastifiant et de la vaseline et la quantité de vaseline, représenté par P+H+V/V, est inférieur à 11 ;
et ledit mélange de 2 copolymères comprend au moins 20 % en poids du premier copolymère.

2. Composition selon la revendication 1 **caractérisé en ce que** la matrice hydrophobe comprend :
- pour 100 parties en poids d'un mélange P de 2 copolymères tribloc du type styrène - oléfine saturée - styrène, un premier qui présente une viscosité comprise entre 0,01 et 1 Pa.s telle que mesurée à 30°C dans une solution à 5 % (masse/masse) dans le toluène et un second qui présente une viscosité comprise entre 0,01 et 0,5 Pa.s mesurée à 30°C dans une solution à 15 % (masse / masse) dans le toluène ;
- de 500 à 1000 parties en poids d'un plastifiant H, de préférence une huile plastifiante ; et
- de 100 à 400 parties en poids de vaseline V ;
étant en outre précisé que :
- la quantité totale, représentée par P+H+V, de mélange d'élastomères, du plastifiant et de la vaseline est comprise entre 700 et 1200 parties en poids ;
- le rapport entre la quantité totale du mélange d'élastomères, du plastifiant et de la vaseline et la quantité de vaseline, représenté par P+H+V/V, est inférieur à 8 ;
et ledit mélange de 2 copolymères comprend au moins 20 % en poids du premier copolymère.

3. Composition selon l'une des revendications précédentes, **caractérisée** en ce le mélange de 2 copolymères comprend au moins 50% du copolymère qui présente une viscosité comprise entre 0,01 et 1 Pa.s, et en ce que ce mélange est constitué de préférence de 70% en poids du copolymère qui présente une viscosité comprise entre 0,01 et 1 Pa.s et de 30 % en poids du copolymère qui présente une viscosité comprise entre 0,01 et 0,5 Pa.s.

4. Composition selon l'une des revendications précédentes, **caractérisée en ce que** la matrice hydrophobe comprend en outre des particules d'hydrocolloïde en une quantité inférieure ou égale à 25 % en poids, rapportée au poids total de ladite matrice hydrophobe.

5. Composition selon la revendication 4, **caractérisée en ce que** les particules d'hydrocolloïde sont incorporées dans la matrice hydrophobe en une quantité comprise entre 2 et 20% et de préférence entre 10 et 15 % en poids, rapportée au poids total de ladite matrice hydrophobe.

6. Composition selon l'une des revendications précédentes, **caractérisée en ce qu'**elle comprend, en outre, une ou plusieurs substances choisies parmi les substances ayant un rôle favorable dans le traitement des plaies, les agents bactéricides ou bactériostatiques, les agents anti-douleurs ou les anesthésiques locaux ainsi que les agents anti-inflammatoires, en une quantité comprise entre 0,01 et 20 % en poids, de préférence entre 1 et 15 % en poids, rapportée au poids total de la composition.

7. Pansement interface avec armature, avec support ou autoporté, **caractérisé en ce qu'**il comprend une composition selon l'une des revendications 1 à 6.

8. Pansement interface autoporté formé d'une couche mince d'une composition comprenant une matrice hydrophobe et comportant des trous traversants, **caractérisé en ce que** ladite matrice hydrophobe comprend :
- pour 100 parties en poids d'un mélange P de 2 copolymères tribloc du type styrène - oléfine saturée - styrène, un premier qui présente une viscosité comprise entre 0,01 et 1 Pa.s telle que mesurée à 30°C dans une solution à 5 % (masse/masse) dans le toluène et un second qui présente une viscosité comprise entre 0,01 et 0,5 Pa.s mesurée à 30°C dans une solution à 15 % (masse/masse) dans le toluène ;
- de 500 à 1000 parties en poids d'un plastifiant H, de préférence une huile plastifiante ; et
- de 100 à 400 parties en poids de vaseline V ;
étant en outre précisé que :
- la quantité totale, représentée par P+H+V, de mélange d'élastomères, du plastifiant et de la vaseline est comprise entre 700 et 1200 parties en poids ;
- le rapport entre la quantité totale du mélange d'élastomères, du plastifiant et de la vaseline et la quantité de vaseline, représenté par P+H+V/V, est inférieur à 8 ;
et ledit mélange de 2 copolymères comprend au moins 30 % en poids du premier copolymère.

9. Pansement interface autoporté selon la revendication 8, **caractérisé en ce que** la matrice hydrophobe comprend en outre des particules d'hydrocolloïde en une quantité inférieure ou égale à 25 % en poids, rapportée au poids total de ladite matrice hydrophobe.

10. Pansement interface autoporté selon l'une des revendications 8 ou 9, **caractérisé en ce que** la matrice hydrophobe comprend :
- pour 100 parties en poids d'un mélange P de 2 copolymères tribloc du type styrène - oléfine saturée - styrène, un premier qui présente une viscosité comprise entre 0,01 et 1 Pa.s telle que mesurée à 30°C dans une solution à 5 % (masse/masse) dans le toluène et un second qui présente une viscosité comprise entre 0,01 et 0,5 Pa.s mesurée à 30°C dans une solution à 15 % (masse/masse) dans le toluène ;
- de 650 à 800 parties en poids d'un plastifiant H, de préférence une huile plastifiante ; et
- de 150 à 200 parties en poids de vaseline V ;
étant en outre précisé que :
- la quantité totale, représentée par P+H+V, de mélange d'élastomères, du plastifiant et de la vaseline est comprise entre 900 et 1100 parties en poids;
- le rapport entre la quantité totale du mélange d'élastomères, du plastifiant et de la vaseline et la quantité de vaseline, représenté par P+H+V/V, est inférieur à 6 ;
ledit mélange de 2 copolymères comprend au moins 50 % en poids du premier polymère
- et des particules d'hydrocolloïdes en une quantité comprise entre 10 et 20 % en poids rapportée au poids total de la matrice hydrophobe.

11. Pansement interface autoporté selon l'une des revendications 8 à 10, **caractérisé en ce qu'**il se présente sous la forme d'un filet aéré dont la taille de maille est de l'ordre de 2 mm, l'épaisseur de l'ordre de 600 microns et le grammage de l'ordre de 450 g/m².

## Patentansprüche

1. Zusammensetzung, die eine geringe Affinität gegenüber Latexhandschuhen aufweist, umfassend eine hydrophobe Matrix, **dadurch gekennzeichnet, dass** die hydrophobe Matrix umfasst:
- bei 100 Gewichtsteilen einer Mischung P aus 2 Triblockcopolymeren vom Typ Styrol - gesättigtes Olefin - Styrol, ein erstes, das eine Viskosität im Bereich zwischen 0,01 und 1 Pa.s aufweist, gemessen bei 30 °C in einer 5%igen (w/w) Lösung in Toluol, und ein zweites, das eine Viskosität im Bereich zwischen 0,01 und 0,5 Pa.s aufweist, gemessen bei 30 °C in einer 15%igen (w/w) Lösung in Toluol,
- 300 bis 1000 Gewichtsteile eines Weichmachers H, vorzugsweise eines Weichmacheröls; und
- 90 bis 600 Gewichtsteile Vaseline V,
wobei weiterhin präzisiert ist, dass:
- die Gesamtmenge, dargestellt durch P+H+V, der Mischung aus Elastomeren, des Weichmachers und der Vaseline im Bereich zwischen 490 und 1700 Gewichtsteilen liegt,
- das Verhältnis zwischen der Gesamtmenge der Mischung aus Elastomeren, des Weichmachers und der Vaseline und der Menge der Vaseline, dargestellt durch P+H+V/V, weniger als 11 beträgt,
und die Mischung aus 2 Copolymeren wenigstens 20 Gew.-% des ersten Copolymers umfasst.

2. Zusammensetzung nach Anspruch 1, **dadurch gekennzeichnet, dass** die hydrophobe Matrix umfasst:
- bei 100 Gewichtsteilen einer Mischung P aus 2 Triblockcopolymeren vom Typ Styrol - gesättigtes Olefin - Styrol, ein erstes, das eine Viskosität im Bereich zwischen 0,01 und 1 Pa.s aufweist, gemessen bei 30 °C in einer 5%igen (w/w) Lösung in Toluol, und ein zweites, das eine Viskosität im Bereich zwischen 0,01 und 0,5 Pa.s aufweist, gemessen bei 30 °C in einer 15%igen (w/w) Lösung in Toluol,
- 500 bis 1000 Gewichtsteile eines Weichmachers H, vorzugsweise eines Weichmacheröls; und
- 100 bis 400 Gewichtsteile Vaseline V,
wobei weiterhin präzisiert ist, dass:
- die Gesamtmenge, dargestellt durch P+H+V, der Mischung aus Elastomeren, des Weichmachers und der Vaseline im Bereich zwischen 700 und 1200 Gewichtsteilen liegt,
- das Verhältnis zwischen der Gesamtmenge der Mischung aus Elastomeren, des Weichmachers und der Vaseline und der Menge der Vaseline, dargestellt durch P+H+V/V, weniger als 8 beträgt,
und die Mischung aus 2 Copolymeren wenigstens 20 Gew.-% des ersten Copolymers umfasst.

3. Zusammensetzung nach einem der vorhergehenden Ansprüche, **dadurch gekennzeichnet, dass** die Mischung aus 2 Copolymeren wenigstens 50 % des Copolymers umfasst, das eine Viskosität im Bereich zwischen 0,01 und 1 Pa.s aufweist, und dass diese Mischung aus vorzugsweise 70 Gew.-% des Copolymers, das eine Viskosität im Bereich zwischen 0,01 und 1 Pa.s aufweist, und 30 Gew.-% des Copolymers, das eine Viskosität im Bereich zwischen 0,01 und 0,5 Pa.s aufweist, besteht.

4. Zusammensetzung nach einem der vorhergehenden Ansprüche, **dadurch gekennzeichnet, dass** die hydrophobe Matrix ferner Hydrokolloidpartikel in einer Menge von weniger als oder gleich 25 Gew.-%, bezogen auf das Gesamtgewicht der hydrophoben Matrix, umfasst.

5. Zusammensetzung nach Anspruch 4, **dadurch gekennzeichnet, dass** die Hydrokolloidpartikel in einer Menge zwischen 2 und 20 und vorzugsweise zwischen 10 und 15 Gew.-%, bezogen auf das Gesamtgewicht der hydrophoben Matrix, in die hydrophobe Matrix eingebettet sind.

6. Zusammensetzung nach einem der vorhergehenden Ansprüche, **dadurch gekennzeichnet, dass** sie weiterhin eine oder mehrere Substanzen enthält, ausgewählt aus Substanzen mit einer günstigen Rolle bei der Wundbehandlung, Bakteriziden oder Bakteriostatika, schmerzstillenden Mitteln oder Lokalanästhetika sowie entzündungshemmenden Mitteln, in einer Menge zwischen 0,01 und 20 Gew.-%, vorzugsweise zwischen 1 und 15 Gew.-%, bezogen auf das Gesamtgewicht der Zusammensetzung.

7. Wundauflage mit Einlage, mit Träger oder selbsttragend, **dadurch gekennzeichnet, dass** sie eine Zusammensetzung nach einem der Ansprüche 1 bis 6 enthält.

8. Selbsttragende Wundauflage, die aus einer dünnen Schicht einer Zusammensetzung, welche eine hydrophobe Matrix aufweist, gebildet ist und Durchgangslöcher umfasst, **dadurch gekennzeichnet, dass** die hydrophobe Matrix umfasst:
- bei 100 Gewichtsteilen einer Mischung P aus 2 Triblockcopolymeren vom Typ Styrol - gesättigtes Olefin - Styrol, ein erstes, das eine Viskosität im Bereich zwischen 0,01 und 1 Pa.s aufweist, gemessen bei 30 °C in einer 5%igen (w/w) Lösung in Toluol, und ein zweites, das eine Viskosität im Bereich zwischen 0,01 und 0,5 Pa.s aufweist, gemessen bei 30 °C in einer 15%igen (w/w) Lösung in Toluol,
- 500 bis 1000 Gewichtsteile eines Weichmachers H, vorzugsweise eines Weichmacheröls; und
- 100 bis 400 Gewichtsteile Vaseline V,
wobei weiterhin präzisiert ist, dass:
- die Gesamtmenge, dargestellt durch P+H+V, der Mischung aus Elastomeren, des Weichmachers und der Vaseline im Bereich zwischen 700 und 1200 Gewichtsteilen liegt,
- das Verhältnis zwischen der Gesamtmenge der Mischung aus Elastomeren, des Weichmachers und der Vaseline und der Menge der Vaseline, dargestellt durch P+H+V/V, weniger als 8 beträgt,
und die Mischung aus 2 Copolymeren wenigstens 30 Gew.-% des ersten Copolymers umfasst.

9. Selbsttragende Wundauflage nach Anspruch 8, **dadurch gekennzeichnet, dass** die hydrophobe Matrix ferner Hydrokolloidpartikel in einer Menge von weniger als oder gleich 25 Gew.-%, bezogen auf das Gesamtgewicht der hydrophoben Matrix, umfasst.

10. Selbsttragende Wundauflage nach einem der Ansprüche 8 oder 9, **dadurch gekennzeichnet, dass** die hydrophobe Matrix umfasst:
- bei 100 Gewichtsteilen einer Mischung P aus 2 Triblockcopolymeren vom Typ Styrol - gesättigtes Olefin - Styrol, ein erstes, das eine Viskosität im Bereich zwischen 0,01 und 1 Pa.s aufweist, gemessen bei 30 °C in einer 5%igen (w/w) Lösung in Toluol, und ein zweites, das eine Viskosität im Bereich zwischen 0,01 und 0,5 Pa.s aufweist, gemessen bei 30 °C in einer 15%igen (w/w) Lösung in Toluol,
- 650 bis 800 Gewichtsteile eines Weichmachers H, vorzugsweise eines Weichmacheröls; und
- 150 bis 200 Gewichtsteile Vaseline V,
wobei weiterhin präzisiert ist, dass:
- die Gesamtmenge, dargestellt durch P+H+V, der Mischung aus Elastomeren, des Weichmachers und der Vaseline im Bereich zwischen 900 und 1100 Gewichtsteilen liegt,
- das Verhältnis zwischen der Gesamtmenge der Mischung aus Elastomeren, des Weichmachers und der Vaseline und der Menge der Vaseline, dargestellt durch P+H+V/V, weniger als 6 beträgt,
die Mischung aus 2 Copolymeren wenigstens 50 Gew.-% des ersten Polymers umfasst.
- und Hydrokolloidpartikel in einer Menge zwischen 10 und 20 Gew.-% bezogen auf das Gesamtgewicht der hydrophoben Matrix.

11. Selbsttragende Wundauflage nach einem der Ansprüche 8 bis 10, **dadurch gekennzeichnet, dass** sie in Form eines durchbrochenen Netzes vorliegt, dessen Maschenweite in der Größenordnung von 2 mm, dessen Dicke in der Größenordnung von 600 µm und dessen Flächengewicht in der Größenordnung von 450 g/m² liegt.

## Claims

1. A composition which has a low affinity with respect to latex gloves, comprising a hydrophobic matrix, **characterized in that** said hydrophobic matrix comprises:
- for 100 parts by weight of a mixture P of 2 styrene-saturated olefin-styrene triblock copolymers, a first which has a viscosity of between 0.01 and 1 Pa.s as measured at 30°C in a 5% (weight/weight) solution in toluene and a second which has a viscosity of between 0.01 and 0.5 Pa.s as measured at 30°C in a 15% (weight/weight) solution in toluene;
- from 300 to 1000 parts by weight of a plasticizer H, preferably a plasticizing oil; and
- from 90 to 600 parts by weight of petroleum jelly V;
it also being specified that:
- the total amount, represented by P+H+V, of the mixture of elastomers, of plasticizer and of petroleum jelly is between 490 and 1700 parts by weight;
- the ratio between the total amount of the mixture of elastomers, of plasticizer and of petroleum jelly and the amount of petroleum jelly, represented by P+H+V/V, is less than 11;
and said mixture of 2 copolymers comprises at least 20% by weight of the first copolymer.

2. The composition as claimed in claim 1, **characterized in that** the hydrophobic matrix comprises:
- for 100 parts by weight of a mixture P of 2 styrene-saturated olefin-styrene triblock copolymers, a first which has a viscosity of between 0.01 and 1 Pa.s as measured at 30°C in a 5% (weight/weight) solution in toluene and a second which has a viscosity of between 0.01 and 0.5 Pa.s as measured at 30°C in a 15% (weight/weight) solution in toluene;
- from 500 to 1000 parts by weight of a plasticizer H, preferably a plasticizing oil; and
- from 100 to 400 parts by weight of petroleum jelly V;
it also being specified that:
- the total amount, represented by P+H+V, of the mixture of elastomers, of plasticizer and of petroleum jelly is between 700 and 1200 parts by weight;
- the ratio between the total amount of the mixture of elastomers, of plasticizer and of petroleum jelly and the amount of petroleum jelly, represented by P+H+V/V, is less than 8;
and said mixture of 2 copolymers comprises at least 20% by weight of the first copolymer.

3. The composition as claimed in either of the preceding claims, **characterized in that** the mixture of 2 copolymers comprises at least 50% of the copolymer which has a viscosity of between 0.01 and 1 Pa.s, and **in that** this mixture preferably consists of 70% by weight of the copolymer which has a viscosity of between 0.01 and 1 Pa.s and of 30% by weight of the copolymer which has a viscosity of between 0.01 and 0.5 Pa.s.

4. The composition as claimed in one of the preceding claims, **characterized in that** the hydrophobic matrix also comprises hydrocolloid particles in an amount of less than or equal to 25% by weight, relative to the total weight of said hydrophobic matrix.

5. The composition as claimed in claim 4, **characterized in that** the hydrocolloid particles are incorporated into the hydrophobic matrix in an amount of between 2% and 20% and preferably between 10% and 15% by weight, relative to the total weight of said hydrophobic matrix.

6. The composition as claimed in one of the preceding claims, **characterized in that** it also comprises one or more substances chosen from substances which have a favorable role in wound treatment, bactericidal or bacteriostatic agents, painkillers or local anesthetics and also anti-inflammatory agents, in an amount of between 0.01% and 20% by weight, preferably between 1% and 15% by weight, relative to the total weight of the composition.

7. An interface dressing with reinforcement, with a support or which is self-supported, **characterized in that** it comprises a composition as claimed in one of claims 1 to 6.

8. A self-supported interface dressing formed from a thin layer of a composition comprising a hydrophobic matrix and comprising through-holes, **characterized in that** said hydrophobic matrix comprises:
- for 100 parts by weight of a mixture P of 2 styrene-saturated olefin-styrene triblock copolymers, a first which has a viscosity of between 0.01 and 1 Pa.s as measured at 30°C in a 5% (weight/weight) solution in toluene and a second which has a viscosity of between 0.01 and 0.5 Pa.s measured at 30°C in a 15% (weight/weight) solution in toluene;
- from 500 to 1000 parts by weight of a plasticizer H, preferably a plasticizing oil; and
- from 100 to 400 parts by weight of petroleum jelly V;
it also being specified that:
- the total amount, represented by P+H+V, of the mixture of elastomers, of plasticizer and of petroleum jelly is between 700 and 1200 parts by weight;
- the ratio between the total amount of the mixture of elastomers, of plasticizer and of petroleum jelly and the amount of petroleum jelly, represented by P+H+V/V, is less than 8;
and said mixture of 2 copolymers comprises at last 30% by weight of the first copolymer.

9. The self-supported interface dressing as claimed in claim 8, **characterized in that** the hydrophobic matrix also comprises hydrocolloid particles in an amount of less than or equal to 25% by weight, relative to the total weight of said hydrophobic matrix.

10. The self-supported interface dressing as claimed in either of claims 8 and 9, **characterized in that** the hydrophobic matrix comprises:
- for 100 parts by weight of a mixture P of 2 styrene-saturated olefin-styrene triblock copolymers, a first which has a viscosity of between 0.01 and 1 Pa.s as measured at 30°C in a 5% (weight/weight) solution in toluene and a second which has a viscosity of between 0.01 and 0.5 Pa.s as measured at 30°C in a 15% (weight/weight) solution in toluene;
- from 650 to 800 parts by weight of a plasticizer H, preferably a plasticizing oil; and
- from 150 to 200 parts by weight of petroleum jelly V;
it also being specified that:
- the total amount, represented by P+H+V, of the mixture of elastomers, of plasticizer and of petroleum jelly is between 900 and 1100 parts by weight;
- the ratio between the total amount of the mixture of elastomers, of plasticizer and of petroleum jelly and the amount of petroleum jelly, represented by P+H+V/V, is less than 6;
said mixture of 2 copolymers comprises at least 50% by weight of the first polymer;
- and hydrocolloid particles in an amount of between 10% and 20% by weight relative to the total weight of the hydrophobic matrix.

11. The self-supported interface dressing as claimed in one of claims 8 to 10, **characterized in that** it is in the form of a breathable net of which the mesh size is about 2 mm, the thickness is about 600 microns and the grammage is about 450 g/m².
